# EUROPEAN PATENT SPECIFICATION

(11) **EP 1 971 395 B1**
(45) Date of publication and mention of the grant of the patent: **18.02.2015**
(21) Application number: 06840352.6
(22) Date of filing: 27.12.2006
(51) Int. Cl.: A61N 1/37, A61N 1/365

(54) **SYSTEM FOR INTERPRETING HEMODYNAMIC DATA INCORPORATING PATIENT POSTURE INFORMATION**
SYSTEM ZUR INTERPRETATION VON HÄMODYNAMISCHEN DATEN MIT INFORMATIONEN ÜBER DIE HALTUNG EINES PATIENTEN
SYSTÈME D'INTERPRÉTATION DE DONNÉES HÉMODYNAMIQUES INTÉGRANT DES INFORMATIONS DE POSTURE DU PATIENT

(30) Priority: 30.12.2005 US 323895
(43) Date of publication of application: 24.09.2008
(73) Proprietor: Medtronic, Inc., Minneapolis, MN 55432-5604 (US)
(72) Inventor: CHO, Yong Kyun, Maple Grove, Minnesota 55369 (US); CHO, Yongduk, Shoreview, Minnesota 55126 (US); BENNETT, Tommy D., Shoreview, Minnesota 55126 (US)
(74) Representative: Hughes, Andrea Michelle
(86) International application number: PCT/US2006/062610
(87) International publication number: WO 2007/079376

(56) References cited:
- EP-A- 1 331 022
- EP-A1- 0 845 240
- EP-A2- 0 985 429
- US-A1- 5 299 119
- US-A1- 6 044 297
- US-A1- 2005 043 772
- US-A1- 2005 215 912
- US-B1- 6 308 098

## Description

### FIELD

The present invention relates generally to medical devices, and more particularly to implantable medical devices (IMDs).

### BACKGROUND

The ability to detect patient posture has been demonstrated in the field of implantable medical devices (IMDs). Patient posture information has been used as an input to cardiac pacemakers, for example, to employ a different pacing rate depending on whether the patient is upright or lying down. Gravity switches, accelerometers, and pressure sensors are among the types of sensors that have been proposed as posture sensors. Such IMDs are known in the art, for example, EP 1331022 discloses an implantable medical device for monitoring physiological signals.

Hemodynamic parameters of a patient, such as right ventricular pressure (RVP), left ventricular pressure (LVP), and estimated pulmonary arterial diastolic pressure (ePAD), may be recorded and monitored on an ambulatory basis to provide information to a physician to facilitate diagnosis and treatment. Abnormal patterns or changes in a patient's recorded hemodynamic parameters may indicate a pathophysiologic change, and may provide the basis for prescribing certain treatment regimens, such as drug or device therapies, for example.

Ambulatory hemodynamic monitoring may be performed by an implantable hemodynamic monitor (IHM), which may possess the ability to record and store a number of hemodynamic parameters, such as intracardiac blood pressures and related parameters. IHMs that record intracardiac electrogram (EGM) signals from electrodes placed in or about the heart, as well as other physiologic sensor derived signals, e.g., one or more of blood pressure, blood gases, temperature, electrical impedance of the heart and/or chest, and patient activity, have been proposed for use in IHMs. An IHM may, for example, be coupled to a lead having capacitive blood pressure and temperature sensors as well as EGM sense electrodes. Such implantable monitors, when implanted in patients suffering from cardiac arrhythmias or heart failure, may accumulate date-and time-stamped data that can be of use in determining the condition of the heart over an extended period of time, including while the patient is engaged in daily activities.

The results of ambulatory hemodynamic monitoring may be presented to a physician in a summarized numerical form, such as a series of daily median values and/or night-time minimum values, which may be provided for any or all of the monitored hemodynamic parameters. A physician may compare such summary data values to measured "baseline" values, taken during an earlier hospital or office visit, for example, to form the basis for therapy decisions, or to make changes to a current treatment. Physicians may expect to see summary data values that correlate with the data measured in the clinical setting, which typically includes only one "recumbent" posture (e.g., usually supine).

US 5,299,119 relates to an instrument which non-invasively monitors EKG information in relationship to the respiratory cycle and/or postural changes.

EP 0985429 relates to a device for assessing the degree of heart failure in a patient comprising means for sensing the posture of the patient.

EP1331022 relates to an implantable medical system that analyses a body position to determine if a vasovagal syncope is indicated.

US2005/0215912 relates to a blood pressure cuff and correcting the measured blood pressure based on a measured position of the arm.

### SUMMARY OF THE INVENTION

In certain embodiments of the invention, an implantable medical device system (claim 1) for interpreting hemodynamic data which incorporates patient posture information is disclosed which includes acquiring hemodynamic data, acquiring a posture signal, classifying the posture signal, categorizing hemodynamic data according to the posture classification, and interpreting hemodynamic data acquired in one posture classification differently from hemodynamic data acquired in other posture classifications. Certain embodiments may include normalizing hemodynamic data according to the classified patient posture, so that the effects of changes in posture are accounted for in the interpretation of data obtained in a number of different patient postures.

In certain embodiments of the invention, a system for interpreting hemodynamic data which incorporates patient posture information is disclosed which includes establishing baseline hemodynamic "profiles" for a number of different postures from which ambulatory hemodynamic data may be classified according to posture, and interpreting the acquired ambulatory hemodynamic data using the derived posture information.

### DESCRIPTION OF THE DRAWINGS

FIG. 1 is a schematic diagram depicting a multi-channel, atrial and bi-ventricular, monitoring/pacing implantable medical device (IMD) system in which certain embodiments of the invention may be implemented.
FIG. 2 is a block diagram of one embodiment of IMD circuitry and associated leads employed in the system of FIG. 1 enabling selective therapy delivery and hemodynamic parameter monitoring in one or more heart chambers.
FIG. 3 is a block diagram of a single monitoring and pacing channel for deriving pressure impedance and cardiac EGM signals in accordance with the present invention,
FIG. 4 is a plot of a hemodynamic pressure parameter as a function of time recorded from a patient with an IHM/IMD during a period of time in which the patient is recumbent (e.g., lying in bed).
FIG. 5 is a distribution plot showing the relative frequency of occurrence of acquired hemodynamic pressure data as a function of the measured pressure in accordance with an embodiment of the invention.
FIG. 6 is a distribution plot showing the relative frequencey of occurrence of acquired hemodynamic pressure data as a function of the measured pressure in accordance with an embodiment of the invention.
FIG. 7 is a flow chart describing a method of acquiring and interpreting hemodynamic data in accordance with embodiments of the invention wherein posture information is acquired with the acquisition of hemodynamic data.
FIG. 8 is a flow chart describing a method of acquiring and interpreting hemodynamic data in accordance with embodiments of the invention wherein postural effects on hemodynamic pressure data are acquired from a series of known patient postures to establish baseline or reference values for comparison with subsequently acquired hemodynamic data.

### DETAILED DESCRIPTION

The following detailed description should be read with reference to the drawings, in which like elements in different drawings are numbered identically. The drawings depict selected embodiments and are not intended to limit the scope of the invention. It will be understood that embodiments shown in the drawings and described below are merely for illustrative purposes, and are not intended to limit the scope of the invention as defined in the claims.

The to monitor and measure hemodynamic parameters, such blood pressure, in an ambulatory patient has been demonstrated. Implantable hemodynamic monitors (IMMs), for example, are able to record intra-cardiac blood pressure parameters, as well as other parameters related to cardiac function. IHMs provide the ability to monitor and record hemodynamic data over relatively long periods of time, enabling the measurement of such data during the full range of daily activities of a patient. Such data may be useful to a physician for example, in monitoring a patient's disease progression, or response to pharmacological therapy, for example.

In patients with ambulatory hemodynamic monitoring, changes in patient posture or body position can have measurable effects on measured hemodynamic data that may be unrelated to a pathophysiologic change. Posture-related effects on measured hemodynamic parameters may therefore present confusing data interpretation problems for the physician if not correlated with patient posture.

Changes in the posture of a patient have been observed to cause sudden shifts in the amplitude of measured hemodynamic data. In a recent sleep study, right ventricular pressure (RVP) typically increased about 5-15 mm Hg when patients changed posture from a supine position (i.e., lying flat on their backs) to either a right or left lateral decubitus position (i.e., lying on their right or left sides, respectively).

Various embodiments or the invention, described herein, incorporate the effects of changes in patient posture into the interpretation of hemodynamic data, which may help a physician differentiate such postural effects on acquired hemodynamic data from pathophysiologic changes.

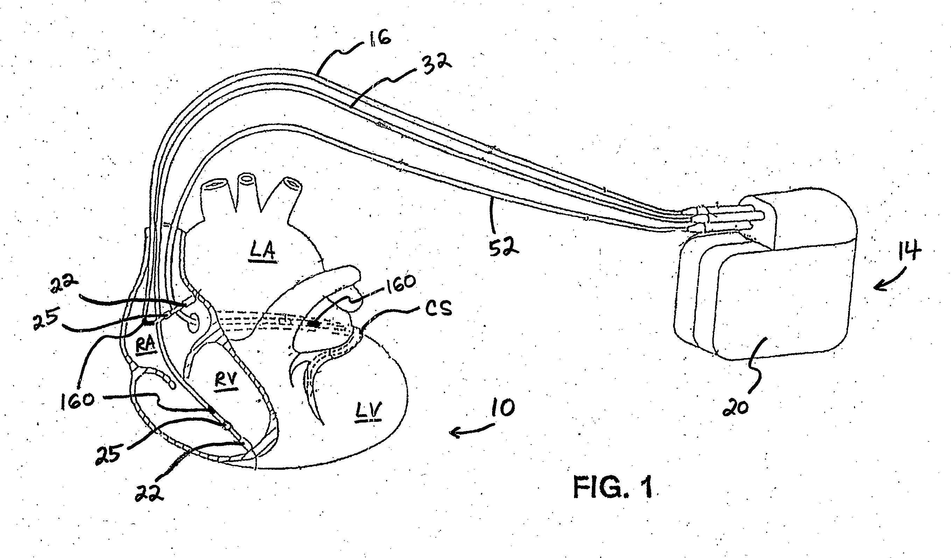

FIG. 1 is a schematic representation of an implantable medical device (IMD) 14 that may be used in accordance with certain embodiments of the invention. The IMD 14 may be any device that is capable of measuring hemodynamic parameters (e.g., blood pressure signals) from within a patient's heart, and which may further be capable of measuring and recording other signal, such as the patient's electrogram (EGM).

In FIG. 1, heart 10 incudes the right atrium (RA), left atrium (LA), right ventricle (RV), left ventricle (LV), and the coronary sinus (CS) extending from the opening in the right atrium laterally around the atria to forum the great vein.

FIG. 1 depicts IMD 14 in reflation to heart 10. In certain embodiments, IMD 14 may be an implantable, multi-channel cardiac pacemaker that may be used for restoring AV synchronous contractions of the atrial and ventricular chambers and simultaneous or sequential pacing of the right and left ventricles. Three endocardial leads 16, 32 and 52 connect the IMD 14 with the RA, the RV and the LV, respectively. Each lead has at least one electrical conductor and pace/sense electrode, and a can electrode 20 may be formed as part of the outer surface of the housing of the HMD 14, The pace/sense electrodes and can electrode 20 may be selectively employed to provide a number of unipolar and bipolar pace/sense electrode combinations for pacing and sensing functions. The depicted positions in or about the right and left heart chambers are merely exemplary. Moreover other leads and pace/sense electrodes may be used instead of the depicted leads and pace/sense electrodes.

In certain other embodiments, IMD 14 may be an implantable hemodynamic monitor (IHM), or an implantable cardioverter defibrillator (ICD), or a cardiac resynchronization therapy (CRT) devices, or any other device that may be adapted to acquire ambulatory hemodynamic data from a patient. Other devices such as implantable drag delivery devices may also be adapted for use with certain embodiments of the invention.

With continued reference to FIG. 1, leads 16, 32, and 52 may be passed through a vein into various chambers of the heart 10. The distal ends of the leads may include tip electrode 22 adapted to make contact with various areas within the heart 10. A unipolar configuration may comprise sensing and/or pacing between a tip electrode 22 and can electrode 20, for example. In a multi-polar configuration, a second ring electrode 25 may be spaced from the tip electrode 22, as is known in the art.

Typically, in pacing systems of the type illustrated in FIG. 1, the electrodes designated above as "pace/sense" electrodes are used for both pacing and sensing functions. In accordance with one aspect of the present invention, these "pace/sense" electrode can be selected to be used exclusively as pace or sense electrodes or to be used in common as pace/sense electrodes in programmed combinations for sensing cardiac signal and delivering pace pulses along pacing and sending vectors.

In addition, some or all of the leads shown in FIG. 1 could carry one or more pressure sensors 160 for developing systolic and diastolic pressures, and a series of spaced apart impedance sensing leads (not shown) for developing volumetric measurements of the expansion and contraction of the RA, LA, RV and LV. If desired, additional leads coupled to IMD 14 may be provided to carry pressure sensor 160. The pressure sensors 160 may be positioned within the heart 10 in a location to measure a desired pressure parameter of interest. Pressure sensors may, for example, be placed in the chambers of the heart, such as the RA, RV and LV. As shown in FIG. 1, a pressure sensor may also be placed in or near the coronary sinus and/or the great cardiac vein to obtain hemodynamic pressure data,

The leads and circuitry described above can be employed to record EGM signals, blood pressure signals, and impedance values, among other possible signals, over certain time intervals, The recorded data may be periodically telemetered out to a programmer operated by a physician or other healthcare worker in an uplink telemetry transmission during a telemetry session, for example,

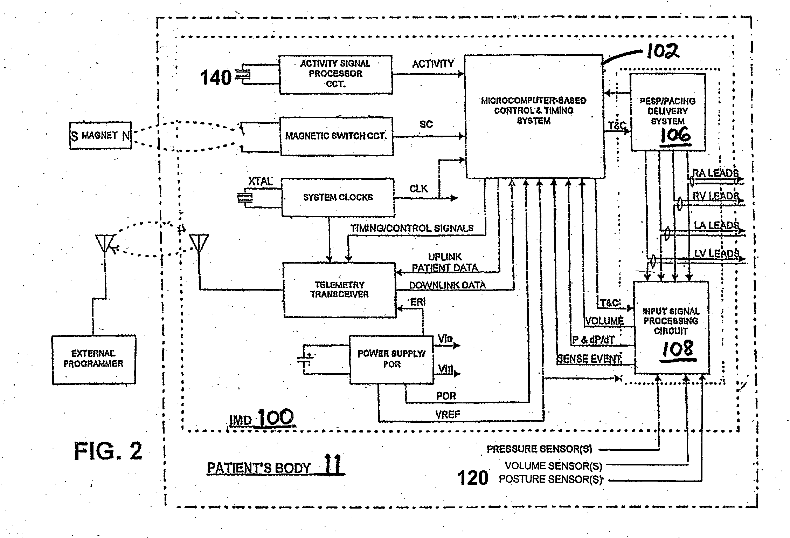

FIG. 2 depicts a system architecture of an exemplary multi-chamber monitor/sensor 100 implanted into a patient's body 11 that provides delivery of a therapy and/or physiologic input signal processing. The typical multi-chamber monitor/sensor 100 has a system architecture that is constructed about a microcomputer-based control and timing system 102 which varies in sophistication and complexity depending upon the type and functional features incorporated therein. The functions of microcomputer-based multi-chamber monitor/sensor control and timing system 102 are controlled by firmware and programmed software algorithms stored in RAM and ROM including PROM and EEPROM and are carried out using a CPU or ALU of a typical microprocessor core architecture. The microcomputer-based multi-chamber monitor/sensor control and timing system 102 may also include a watchdog circuit, a DMA controller, a block mover/reader, a CRC calculator, and other specific logic circuitry coupled together by on-chip data bus, address bus, power, clock, and control signal lines in paths or trees in a manner well known in the art. It will also be understood that control and timing of multi-chamber monitor/sensor 100 can be accomplished with dedicated circuit hardware or state machine logic rather than a programmed micro-computer.

The therapy delivery system 106 can be configured to include circuitry for delivering cardioversion/defibrillation shocks and/or cardiac pacing pulses delivered to the heart or cardiomyostimulation to a skeletal muscle wrapped about the heart. Alternately, the therapy delivery system 106 can be configured as a drug pump for delivering drugs into the heart to alleviate heart failure or to operate an implantable heart assist device or pump implanted in patients awaiting a heart transplant operation.

The input signal processing circuit 108 includes at least one physiologic sensor signal processing channel for sensing and processing a sensor derived signal from a physiologic sensor located in relation to a heart chamber or elsewhere in the body.

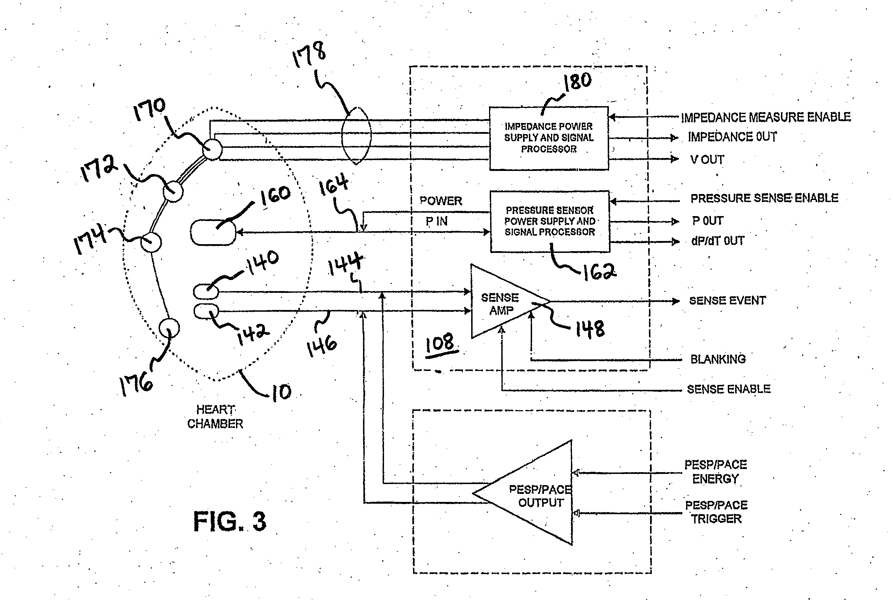

FIG. 3 schematically illustrates one pacing, sensing and parameter measuring channel in relation to one heart chamber. A pair of pace/sense electrodes 140, 142, a pressure sensor 160, and a plurality, e.g., four, impedance measuring electrodes 170, 172, 174, 176 are located in operative relation to the heart 10.

The pair of pace/sense electrodes 140, 142 are located in operative relation to the heart 10 and coupled through lead conductors 144 and 146, respectively, to the inputs of a sense amplifier 148 locates within the input signal processing circuit 108. The sense amplifier 148 is selectively enabled by the presence of a sense enable signal that is provided by control and timing system 102 (FIG. 2). The sense amplifier 148 is enabled during prescribed times when pacing is either enabled or not enabled in a manner known in the pacing art. The blanking signal is provided by control and timing system 102 upon delivery of a pacing or PESP pulse or pulse train to disconnect the sense amplifier inputs from the lead conductors 144 and 146 for a short blanking period in a manner well known in the art. The sense amplifier provides a sense event signal signifying the contraction of the heart chamber commencing a heart cycle based upon characteristics of the EGM. The control and timing system responds to non-refractory sense events by restarting an escape interval (EI) timer timing out the E1 for the heart chamber, in a manner well known in the pacing art.

The pressure sensor 160 may be coupled to a pressure sensor power supply and signal processor 162 within the input signal processing circuit 108 through a set of lead conductors 164. Lead conductors 164 convey power to the pressure sensor 160, and convey sampled blood pressure signals from the pressure sensor 160 to the pressure sensor power supply and signal processor 162. The pressure sensor power supply and signal processor 162 samples the blood pressure impinging upon a transducer surface of the sensor 160 located within the heart chamber when enabled by a pressure sense enable signal from the control and timing system 102. Absolute pressure (P), developed pressure (DP) and pressure rate of change (dP/dt) sample values can be developed by the pressure sensor power supply and signal processor 162 or by the control and timing system 102 for storage and processing.

A variety of hemodynamic parameters may be recorded, for example, including right ventricular (RV) systolic and diastolic pressures (RVSP and RVDP), estimated pulmonary artery diastolic pressure (ePAD), pressure changes with respect to time (dP/dt), heart rate, activity, and temperature. Some parameters may be derived from others, rather than being directly measured. For example, the ePAD parameter may be derived from RV pressures at the moment of pulmonary valve opening, and heart rate may be derived from information in an intracardiac electrogram (EGM) recording,

At set of impedance electrodes 170, 172, 174 and 176 may be coupled by a set of conductors 178 and formed as a lead that is coupled to the impedance power supply and signal processor 180. Impedance-based measurements of cardiac parameters such as stroke volume are known in the art, such as an impedance lead having plural pairs of spaced surface electrodes located within the heart 10. The spaced apart electrodes can also be disposed along impedance leads lodged in cardiac vessels, e.g., the coronary and great vein or attached to the epicardium around the heart chamber. The impedance lead may be combined with the pace/sense and/or pressure sensor bearing lead.

The data stored by IMD 14 may include continuous monitoring of various parameters, for example recording intracardiac EGM data at sampling rates as fast as 256 Hz or faster. In certain embodiments of the invention, an IHM may alternately store summary forms of data that may allow storage of data representing longer periods of time. In one embodiment, hemodynamic pressure parameters may be summarized by storing a number of representative values that describe the hemodynamic parameter over a given storage interval. The mean, median, an upper percentile, and a lower percentile are examples of representative values that my be stored by an IHM to summarise data over an interval of time (e.g., the storage interval). In one embodiment of the invention a storage interval may contain six minutes of data in a data buffer, which may be summarized by storing a median value, a 94th percentile value (i.e., the upper percentile), and a 6th percentile value (i.e., the lower percentile) for each hemodynamic pressure parameter being monitored. In this manner, the memory of the IHM may be able to provide weekly or monthly (or longer) views of the data stored. The data buffer, for example, may acquire data sampled at a 256 Hz sampling rate over a 6 minute storage interval, and the data buffer may be cleared out after the median, upper percentile, and lower percentile values during that 6 minute period are stored. It should be noted that certain parameters measured by the IHM may be summarized by storing fewer values, for example storing only a mean or median value of such parameters as activity level and temperature, according to certain embodiments of the invention.

Hemodynamic parameters that may be used in accordance with various embodiments of the invention include parameters that are directly measured, such as RVDP and RVSP, as well as parameters that may be derived from other pressure parameters, such as estimated pulmonary artery diastolic pressure (ePAD), rate of pressure change (dP/dt), etc.

In some embodiments, IMD 14 may incorporate information from posture sensor(s) 120 to thereby collect patient posture information. In such embodiments, IMD 14 may monitor one or more signals generated by posture sensor(s) 120 that vary as a function of patient posture, and may allow implantable medical device (IMD) 14 to identify or classify a number of postures based on the posture sensor signals. IMD 14 may, for example, periodically identify the posture of a patient, or transitions between postures made by a patient. For example, IMD 14 may identify whether the patient is upright or recumbent (e.g., lying down), and may also identify the timing of transitions between such postures. In certain embodiments of the invention, information from a posture sensor may be used to identify two or more "recumbent" postures and the timing of transitions between such postures. Such recumbent postures may include, for example, supine (i.e., substantially flat on back), left lateral decubitus (i.e., on left side), and right lateral decubitus (i.e., on right side), according to certain embodiments of the invention.

IMD 14 may also incorporate information from an activity sensor 140 to thereby collect patient activity information according to certain embodiments of the invention. Specifically, as will be described in greater detail below, IMD 14 may monitor an activity level of a patient based on an activity signal generated by activity sensor 140 that varies as a function of patient activity. An activity level signal may comprise, for example, a number representative of patient activity (e.g., activity counts). Sensors that output a signal as a function of patient activity may further include one or more bonded piezoelectric crystals, mercury switches, or gyros that generate a signal as a function of body motion, footfalls or other impact events, and the like.

In exemplary embodiments, posture sensor 120 may include two or more accelerometers which are oriented substantially orthogonally with respect to each other. In certain embodiments, three accelerometers (e.g., tri-axial accelerometers) may be used to acquire the desired posture information. In addition to being oriented orthogonally with respect to each other, each of the accelerometers may be substantially aligned with an axis of the body 11 of the patient. The magnitude and polarity of DC components of signals generated by the accelerometers may indicate the orientation of the patient relative to the Earth's gravity, and processor 108 (FIG. 2) may periodically identify the posture or postural changes of the patient based on the magnitude and polarity of the DC components. Further information regarding use of orthogonally aligned accelerometers to determine patient posture may be found in commonly assigned U.S. Pat. No. 5,593,431.

In certain exemplary embodiments, IMD 14 may monitor signals generated by a plurality of accelerometers. In such embodiments, IMD 14 may be adapted to both determine activity levels and identify patient postures (or postural transitions) based on the accelerometer signals.

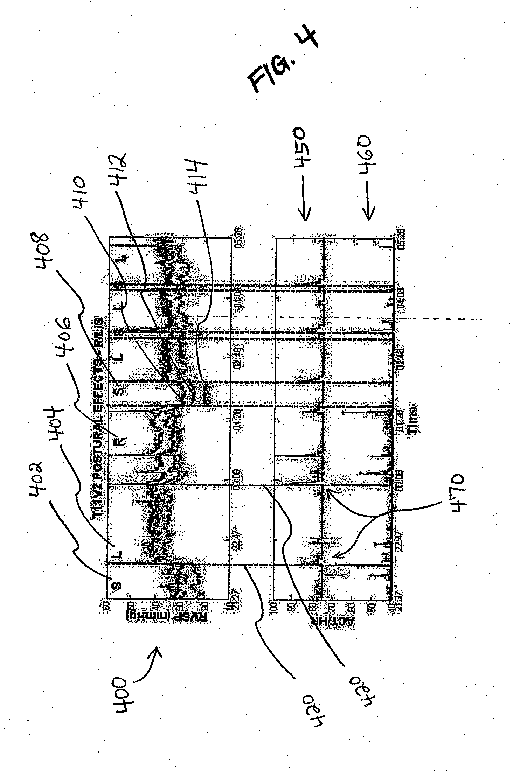

FIG. 4 is a time plot of right ventricular systolic pressure (RVSP) 400 recorded from a patient with an IHM during a period of time in which the patient is in a recumbent posture (e.g., lying in bed). Also plotted in FIG. 4 are the outputs of an activity sensor 460, as well as the patient's heart rate (HR) 450 over the same period of time.

The plot of RVSP 400 shown in FIG. 4 includes three plots of RVSP superimposed on plot 400 to show the range of values that may occur over a period of time. For example, RVSP plot 400 may include an upper range plot 410, a middle range plot 412, and a lower range plot 414 as shown in FIG. 4. As previously described, and in accordance with certain embodiments of the invention, these plots may correspond to predetermined percentile rank values for data acquired during predetermined storage or sampling intervals. For example, upper range plot 410 may represent the 94^{th} percentile value of the RVSP parameter calculated and plotted each storage interval. Similarly, middle range plot 412 and lower range plot 414 may be derived from the 50^{th} percentile (i.e., the median) and 6^{th} percentile values, respectively, of the RVSP parameter. Of course, the particular values chosen are exemplary only and may be varied or adapted as needed. For example, more or less than three plots may be used to represent the range of values that occur over predetermined periods of time. Also, the percentile rank values may be altered to show quartiles, or some other criteria representative of the range of values. Similarly, the storage or sampling interval may be more or less than six minutes, and such modifications would be considered to fall within the scope of the invention as claimed.

In the plot of RVSP 400 in FIG. 4, annotations have been provided to show the patient's posture over the time period indicated. The assessment and classification of patient posture was made by visual observation of a time-correlated video replay of the subject. Patient posture was classified (in this instance) into one of three postures, although other possible posture classification schemes may exist. As shown in FIG. 4, the patient's posture was supine, indicated by "S" 402, during the initial period shown. The patient's posture subsequently sifted to left lateral decubitus, indicated by "L" 404, then to right lateral decubitus, indicated by "R" 406, and so forth.

The timing of shifts in patient posture is also indicated in FIG. 4 by vertical dashed lines 420. As can be seen in the activity sensor and heart rate plots 460, 450, the timing of spikes in the activity sensor signal 460, as indicated at 470 for example, coincided with the visual observance of changes in posture as indicated by dashed lines 420. This would be expected, for example, if the activity sensor was adapted to measure acceleration, vibration, and other forms of energy related to motion of the patient. The sudden increase in the activity sensor signal 460 from a very low lever, and the short duration of the spikes 470, are also consistent with the nature of a change between two recumbent postures, for example.

The plot of RVSP 400 in FIG. 4 indicates that changes in patient posture, even while sleeping (i.e., in a recumbent posture), may cause a noticeable change in measured hemodynamic data. For example, RVSP plot 400 shows that when the patient changed posture from S 402 to L 404, a sudden increase in RVSP is observed that may be on the order of a 5-15 mm Hg pressure difference. A change in RVSP is also noted when the patient's posture changed from a left lateral decubitus posture to a right lateral decubitus posture (from L 404 to R 406), although the magnitude of the difference may be different. A sudden decrease in RVSP is next seen as the patient adjusted posture from R 406 to S408.

From RVSP plot 400, it appears that the range of measured pressures is similar at S 402 and S 408, both being periods of time where the patient was in a supine position. The range of measured parameters while in a given posture may be quantified in a variety of ways to allow for such comparisons. For example, the median (e.g., 50^{th} percentile) plot 412 may be used by itself to provide an indication of the range of a measured hemodynamic parameter, either as a plotted parameter, or reduced to a number (or numbers). For example, the average of the median plot 412 over a period of time, or the high and low values of the median plot 412 over a period of time, may provide useful information to a physician attempting to interpret the data. Alternate measures of the range of measured parameters (i.e., pressures) may incorporate information from the high (e.g., 94^{th} %-ile) plot 410 and the low (e.g., 6^{th} %-ile) plot 414. For example, the average of the low plot 414, when subtracted from the average of the high plot 410, may provide a concise (i.e., a single number) representation of the variability of the measured parameter over a given period of time. Many other forms of data representation may become apparent to one of ordinary skill in the art with the benefit of these teachings, and are considered to be within the scope of the claimed invention.

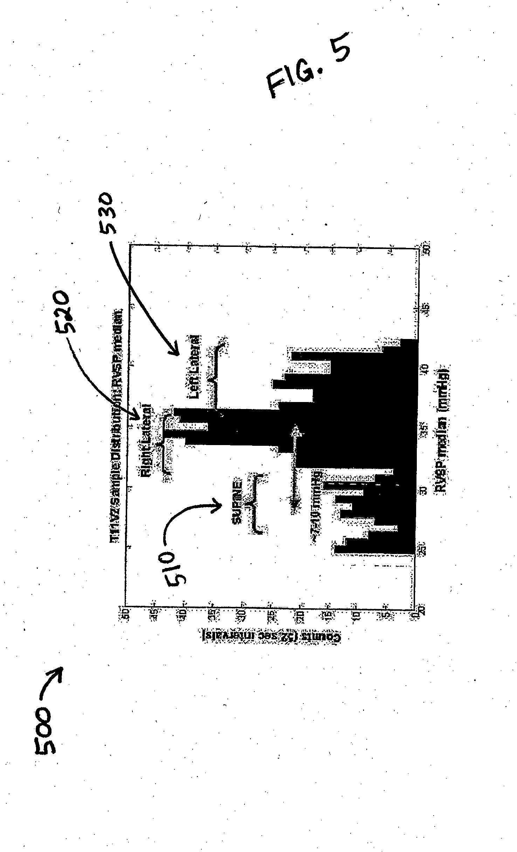

FIG. 5 is a sample distribution plot 500 showing the relative frequency of occurrence of acquired hemodynamic pressure data as a function of the measured pressure. In FIG. 5, RVSP median, which might correspond to a middle range plot of acquired RVSP data (e.g., median plot 412 in Fig. 4), is shown plotted according to the number of occurrences (e.g., "counts," using a 52 second storage internal is FIG. 5) at a particular pressure (or range of pressures). As indicated by distribution plot 500, three relatively distinct "modes" are represented in the plot, corresponding to the three postures observed. For example, a spectrum of measured pressures spanning from about 27 mm Hg to about 32 mm Hg corresponds, to RVSP median values acquired while a patient was in a supine posture, and is labeled supine spectrum 510 in FIG. 5, Similarly, a spectrum of measured pressures spanning from about 32 mm Hg to about 36 mm Hg corresponds to RVSP median values acquired while a patient was in a right lateral decubitus posture (labeled right lateral spectrum 520 in FIG. 5), and a spectrum of measured pressures spanning from about 35 mm Hg to about 43 mm Hg corresponds to RVSP median values acquired while a patient was in a left lateral decubitus posture (left lateral spectrum 530). As indicated by FIG. 5, a significant difference in measured hemodynamic pressures may exist between data acquired while in different postures. In the specific example shown, an increase of approximately 7-10 mm Hg on more is observed to occur when a patient shifts posture from a supine posture to either a left or right lateral decubitus posture. It should be noted that sample distribution 500 shown is exemplary of posture-related shifts in measured hemodynamic data; the magnitude and direction of such posture-related shifts in hemodynamic data may vary from patient to patient.

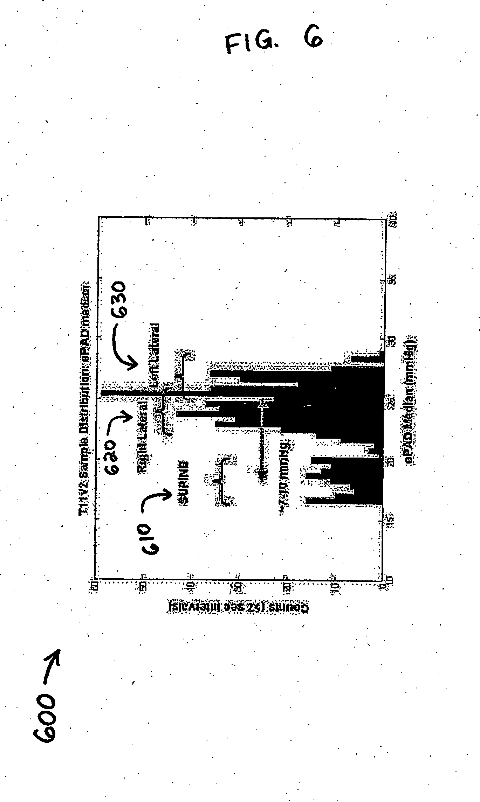

FIG. 6 is a sample distribution plot 600 showing the relative frequency of occurrence of acquired hemodynamic pressure data as a function of the measured pressure. In FIG. 6, estimated pulmonary arterial diastolic pressure (ePAD) median, which might correspond to a middle range plot of acquired (or derived) ePAD data, is shown plotted according to the number of occurrences (e.g., "counts," using a 52 second storage interval in FIG. 6) at a particular pressure (or range of pressures). As shown in FIG. 6, distribution plot 600 displays results similar to those obtained for distribution plot 500 in FIG. 5, including the display of distinct spectra 610, 620, and 630, corresponding to hemodynamic pressure data acquired in different postures. A similar difference of approximately 7-10 mm Hg exists between the supine spectrum 610 and the right and left lateral spectra 620 and 630 for this particular patient as well.

As suggested by FIGS. 5 and 6, posture-related effects on measured hemodynamic data may be similar across a number of different hemodynamic parameters. This may be useful, for example, in using more than one data parameter as the basis for detecting changes in patient posture. An embodiment with no posture sensor, for example, may detect changes in patient posture using changes in hemodynamic data. Two or more parameters may be used to detect or conform a posture change, or a second parameter may be used as a cross-check or confirmation of a posture change detected by a first parameter, for example.

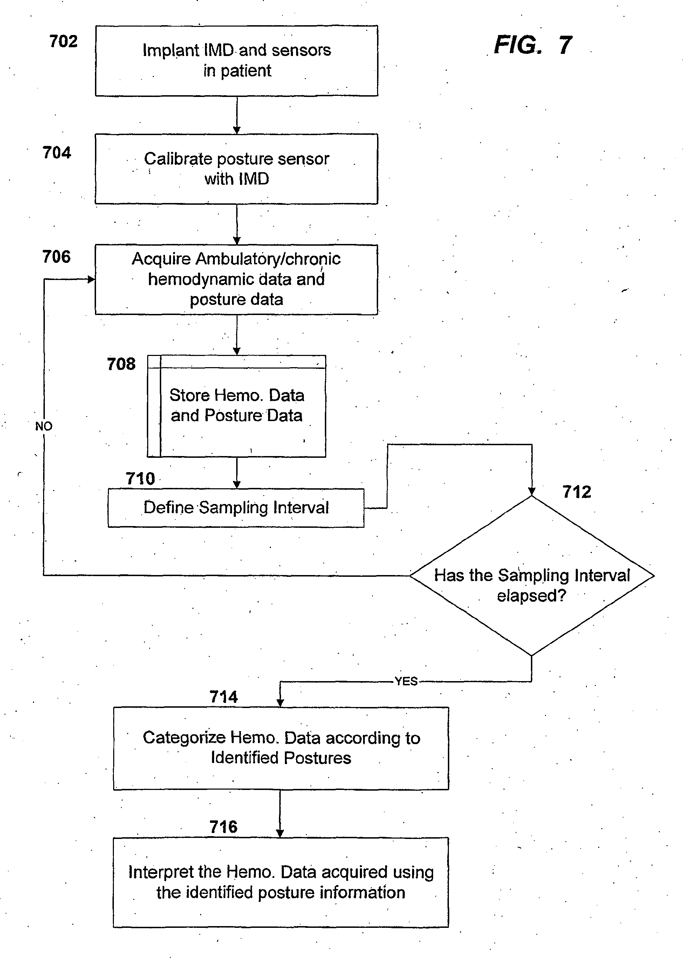

FIG. 7 is a flowchart showing an example of a method of acquiring and interpreting hemodynamic data from a patient wherein information about the patient' s posture is acquired and influenced the interpretation of the hemodynamic data.

The method of acquiring and interpreting hemodynamic data illustrated in FIG. 7 includes step 702, implanting an IMD and sensor system in a patient, As noted previously, an IMD that may be used in accordance with embodiments of the invention may include the ability to monitor hemodynamic parameters, such as hemodynamic pressures, transthoracic impedance, and other desired hemodynamic parameters. Additionally, the IMD may include the ability to receive information from a posture sensor, and may further include the ability to receive information from one or more activity sensors, according to certain embodiments of the invention.

Step 704 includes calibrating a posture sensor with the IMD implanted in step 702. This step enables signals from the posture sensor to be correlated and to correctly identify specific patient postures. The number and type of patient postures used may be determined by an operator (e.g., a physician), and may include upright, supine, and right/left sided positions, for example. It should be noted that step 704 need not be performed at or near the same time as the implantation of IMD in step 702; the calibration of the posture sensor with the IMD may occur at a subsequent patient visit, possibly on an out-patient basis. The next step, step 706, includes acquiring ambulatory data from the patient using the IMD and sensor system implanted in step 702. The ambulatory data acquired may include hemodynamic data (e.g., hemodynamic pressure data), posture signals (e.g., from a posture sensor), activity signals (e.g., from an activity sensor), impedance measurement signals (e.g., transthoracic impedance), or any other suitable data desired to be monitored.

Step 708 in FIG. 7 includes storing hemodynamic data signals and a posture signal in memory. The memory may comprise a memory buffer according to certain embodiments of the invention. The next step, step 710, involves defining or determining a sample interval or storage interval. The sample interval of step 710 may be chosen based on considerations of efficient memory usage. A sample interval, such as 6 minutes, may be chosen in which to perform analysis of the data signals stored during step 708. Step 712 is a decision step that determines whether a sample interval has elapsed. For example, if a sample interval has not elapsed, the method returns to step 706 and continues to acquire ambulatory data from the patient. On the other hand, it a sample interval has elapsed, step 714 may be performed, which includes categorizing hemodynamic data according to the corresponding identified patient posture. Thus, step 714 assigns a specific patient posture to any hemodynamic data acquired.

Step 716 includes interpreting the hemodynamic data acquired using the identified posture associated therewith. In certain embodiments of the invention, the step of interpreting the hemodynamic data based on the identified posture may comprise interpreting hemodynamic data separately, depending on patient posture. For example, in certain embodiments, hemodynamic data acquired while in a supine position may be analyzed or interpreted only in conjunction with other data acquired while in a supine position. Hemodynamic data acquired in a supine posture may, for example, more closely correlate with data collected during implantation of the IMD (assuming a supine posture during implant), or during subsequent follow-up visits, particularly if the patient is observed in a supine position during those scenarios.

In another embodiment of the invention, interpretation of the hemodynamic data based on patient posture may further include calculation of statistics on the percentage on time spent in various patient postures, for example.

In certain other embodiments of the invention, interpretation of hemodynamic data based on patient posture may comprise "normalizing" data taken in various postures to allow for direct comparison between hemodynamic data collected in different postures. For example, if a particular patient exhibits an increase in a hemodynamic pressure parameter of an average of 10 mm Hg when changing from a supine to a left-sided posture, normalization may comprise subtracting 10 mm Hg from hemodynamic pressure data acquired in the affected (e.g., left-sided) posture. In one embodiment, normalization (e.g., adjusting or "correcting") of hemodynamic pressure data acquired while a patient is in right and/or left lateral decubitus postures may allow for meaningful comparison to hemodynamic pressure data acquired in a supine posture, for example. Of course, one of ordinary skill in the art would recognize that other normalization schemes or strategies could be employed to allow for meaningful comparisons between hemodynamic data acquired in different, patient postures. The reference posture, for example, may be chosen to be a right-sided posture if a particular patient spends a significant percentage of their time sleeping in a right-sided posture. Similarly, a measured pressure change from one known posture to another known posture (e.g., from right lateral decubitus to left lateral decubitus) may be used as a diagnostic parameter by monitoring and recording the pressure changes associated with the same type of posture changes over time.

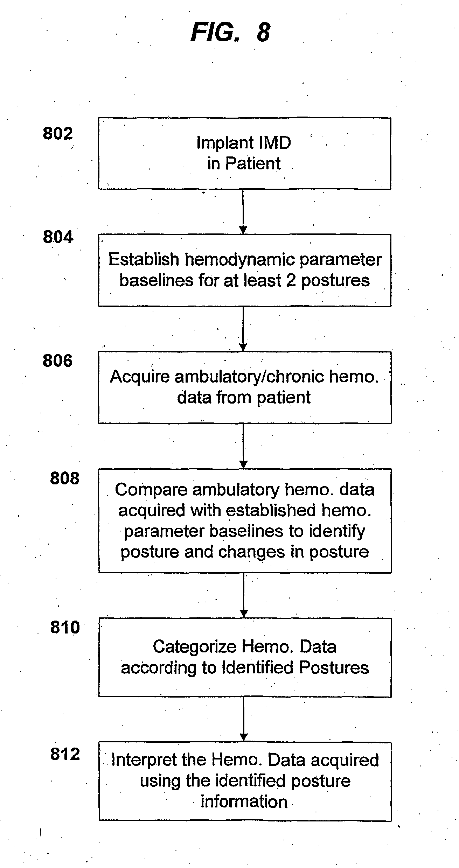

FIG. 8 is a flowchart showing an example of a method of acquiring and interpreting hemodynamic data wherein postural effects may be incorporated without the use of a posture sensor. Hemodynamic data is acquired while a patient assumes a number of known patient postures to establish baseline or reference values (or ranges of values) indicative of each of the known patient postures. Hemodynamic data is subsequently acquired (i.e., on an ambulatory or chronic basis), and may be used to detect changes in patient posture by comparing acquired ambulatory hemodynamic data to the baseline or reference values.

Step 802 comprises implanting an IMD in a patient. The IMD may be adapted to monitor hemodynamic parameters such as pressure, impedance, and other parameters of interest. Step 804 in Fig. 8 includes establishing hemodynamic parameter base lines for at least two patient postures. It should be noted that the step 804 need not be performed at the same time as step 802, and may be performed in a subsequent patient visit, for example, on an out-patient basis. Step 804 may comprise acquiring hemodynamic parameter signals while having the patient assume a number of different known postures. In certain embodiments of the invention, the at least two postures assumed by the patient may include at least two "recumbent" postures, such as supine and either lying on the patient's right or left side, for example, In certain embodiments of the invention, three recumbent postures are used, comprising supine, right sided (right lateral decubitus), and left sided (left lateral decubitus). The establishment of a hemodynamic parameter baseline for each of the designated patient postures may include deriving a cut off value above or bellow which the measured hemodynamic parameter signals will indicate the presence of a particular posture, for example alternately, the hemodynamic parameter baselines may comprise a range of values over which the presence of measured hemodynamic parameter signals would indicate or detect a particular patient posture.

Step 806 includes acquiring ambulatory hemodynamic data from a patient, including data acquired over the normal course of a patient's daily activities. Step 808 comprises comparing the acquired ambulatory hemodynamic data with the hemodynamic parameter baselines established in step 804 to identify postures and changes in posture. Step 810 may next include categorizing the acquired ambulatory hemodynamic data according to the patient posture identified and correlated with such acquired data, for example, Step 812 follows, and includes interpreting the categorized hemodynamic data acquired according to the postures identified in step 808.

In certain embodiments of the invention, step 808 may include using a posture detection criterion (or criteria, if more than one) to identify postures and changes in posture. The posture detection criteria may be based on changes in acutely acquired hemodynamic data signals, for example, a change in a given hemodynamic pressure parameter that exceeds a certain value in a certain period of time. The posture detection criteria may also be based on changes in summary statistical data in certain embodiments. For example, the posture detection criteria may require that a statistical representation (e.g., a median value over a sampling or storage interval) of a hemodynamic pressure parameter change by a predetermined amount and/or fall within a range corresponding to the baseline hemodynamic profiles established for the identified postures to detect a change in patient posture.

In certain further embodiments of the invention, an activity sensor signal may be used in conjunction with the posture signals to identify the timing of changes in posture between two or more recumbent postures. An activity sensor signal of a certain amplitude and duration may, for example, be used to confirm a change in posture detected using either a posture sensor, or using the above-described posture detection method. In some embodiments, the activity sensor signal may increase temporarily from a relatively low activity level, above some predetermined level, and black to a relatively low activity level to confirm a change in posture detected from other means. In further embodiments, the temporary increase in activity level may be required to be complete within a predetermined period of time.

In certain other embodiments of the invention, measured heart rate information may be used to confirm a change in posture detected by other means. For example, a measured heart rate temporarily increasing from below a relatively low rate, above some predetermined heart rate, and back to a relatively low rate can be used to confirm a change in posture detected by other means. In a similar further embodiment, the temporary increase in heart rate may be required to be complete within a predetermined period of time.

Thus, embodiments of a SYSTEM FOR INTERPRETING HEMODYNAMIC DATA INCORPORATING PATIENT POSTURE INFORMATION are disclosed. One skilled in the art will appreciate that the invention can be practiced with embodiments other than those disclosed. The disclosed embodiments are presented for purposes of illustration and not limitation, and the invention is limited only by the claims that follow.

## Claims

1. An implantable medical device system (14) comprising:
means (100) for acquiring a hemodynamic data signal from a patient;
a posture sensor (120) adapted to generate a posture signal;
means (108) for identifying one of at least two patient postures from the posture signal at a given point in time;
means (180) for categorizing the acquired hemodynamic data signal according to the identified posture;
means (102) for storing the categorized hemodynamic data, whereby the stored categorized hemodynamic data facilitates interpretation of the stored hemodynamic data;
**characterized in** further comprising
normalization means for normalizing the hemodynamic data acquired based upon the identified posture, the normalization means comprising
means for applying an adjustment to the hemodynamic data based upon the identified posture to facilitate comparisons of hemodynamic data acquired in different postures.

2. A system according to claim 1 wherein storing hemodynamic data based upon the identified posture comprises storing hemodynamic data acquired in one identified posture separately from hemodynamic data acquired in other identified postures.

3. A system according to claim 1 further comprising means for determining a percentage of time spent in each of the identified postures.

4. A system according to claim 1 wherein the identified postures comprise a supine and at least one lateral decubitus posture.

5. A system according to claim 1 further comprising an activity sensor (460) adapted to generate an activity signal that may be used to identify changes in posture between two distinct postures.

6. A system according to claim 5 wherein the activity signal may be used in conjunction with the posture signal to identify changes in posture between two distinct recumbent postures.

7. A system according to claim 6 wherein a temporary increase in the activity sensor signal from a relatively low activity signal is used to confirm a change in posture between two distinct recumbent postures.

8. A system according to claim 1 further comprising means for measuring the patient's heart rate, wherein a temporary increase in the measured heart rate from a relatively low rate is used to confirm a change in posture between two distinct recumbent postures.

9. A computer-readable medium comprising instructions that, when run on a system as claimed in any preceding claim, cause the system to:
acquire a hemodynamic pressure signal as a function of time;
detect a change in the acquired hemodynamic pressure signal;
associate the change in the hemodynamic pressure signal with a change in patient posture;
store data about the hemodynamic pressure signal and patient posture; and
account for the effects of patient posture in the storage of hemodynamic pressure signal normalizing the hemodynamic data acquired based upon the identified posture, by applying an adjustment to the hemodynamic data based upon the identified posture to facilitate comparisons of hemodynamic data acquired in different postures.

10. A computer-readable medium according to claim 9 adapted to account for the effects of patient posture by storing hemodynamic pressure data obtained in one posture separately from hemodynamic pressure data obtained in another posture.

11. A computer-readable medium according to claim 9 adapted to detect the change in the acquired hemodynamic pressure signal based on a change in pressure greater than a predetermined pressure threshold.

12. A computer-readable medium according to claim 11 wherein the change in pressure greater than a predetermined pressure threshold must also occur within a predetermined time interval.

13. A computer-readable medium according to claim 12 wherein the predetermined pressure threshold is at least 5 mm HG and the predetermined time interval is less than 5 minutes.

## Patentansprüche

1. Implantierbares medizinisches Vorrichtungssystem (14), mit:
Mitteln (100) zum Erfassen eines hämodynamischen Datensignals eines Patienten;
einem Lagesensor (120), der ausgebildet ist, um ein Haltungssignal zu erzeugen;
Mitteln (108) zum Identifizieren einer von zumindest zwei Haltungen des Patienten aus dem Lagesignal an einem gegebenen Zeitpunkt;
Mitteln (180) zum Kategorisieren des erfassten hämodynamischen Datensignals gemäß der identifizierten Haltung; und
Mitteln (102) zum Speichern der kategorisierten hämodynamischen Daten, wobei die gespeicherten kategorisierten hämodynamischen Daten eine Interpretation der gespeicherten hämodynamischen Daten erleichtern;
**gekennzeichnet durch**
Normalisierungsmittel zum Normalisieren der hämodynamischen Daten, die basierend auf der identifizierten Lage erfasst wurden, wobei die Normalisierungsmittel Mittel zum Durchführen einer Einstellung der hämodynamischen Daten basierend auf der identifizierten Haltung aufweisen, um Vergleiche von hämodynamischen Daten zu erleichtern, die für verschiedene Haltungen erfasst wurden.

2. System nach Anspruch 1, wobei das Speichern von hämodynamischen Daten basierend auf der identifizierten Haltung das Speichern hämodynamischer Daten, die bei einer identifizierten Haltung erfasst werden, getrennt von hämodynamischen Daten, die bei anderen identifizierten Haltungen erfasst wurden, umfasst.

3. System nach Anspruch 1, ferner mit Mitteln zum Bestimmen eines Prozentsatzes der Zeit, die in jeder der identifizierten Haltungen verbracht wurde.

4. System nach Anspruch 1, wobei die identifizierten Haltungen eine Rücken- und zumindest eine Dekubitus-Haltung umfassen.

5. System nach Anspruch 1, ferner mit einem Aktivitätssensor (460), der ausgebildet ist, ein Aktivitätssignal zu erzeugen, das verwendet werden kann, um Änderungen in der Haltung zwischen zwei unterschiedlichen Haltungen zu identifizieren.

6. System nach Anspruch 5, wobei das Aktivitätssignal in Verbindung mit dem Haltungssignal verwendet werden kann, um Änderungen in der Haltung zwischen zwei unterschiedlichen Liegehaltungen zu identifizieren.

7. System nach Anspruch 6, wobei ein vorübergehender Anstieg des Aktivitätssensorsignals von einem relativ kleinen Aktivitätssignal verwendet wird, um eine Änderung der Haltung zwischen zwei unterschiedlichen Liegehaltungen zu bestätigen.

8. System nach Anspruch 1, ferner mit Mitteln zum Messung der Herzfrequenz des Patienten, wobei ein vorübergehender Anstieg der gemessenen Herzfrequenz von einer relativ kleinen Frequenz verwendet wird, um eine Änderung der Haltung zwischen zwei unterschiedlichen Liegehaltungen zu bestätigen.

9. Computerlesbares Medium, mit Anweisungen, die auf einem System nach einem der vorstehenden Ansprüche ablaufend das System veranlassen,
ein hämodynamisches Drucksignal als Funktion der Zeit zu erfassen;
eine Änderung des erfassten hämodynamischen Drucksignals zu detektieren;
die Änderung des hämodynamischen Drucksignals mit einer Änderung der Haltung des Patienten zu assoziieren;
Daten des hämodynamischen Drucksignals und der Haltung des Patienten zu speichern; und
die Effekte der Haltung de Patienten bei der Speicherung des hämodynamischen Drucksignals zu berücksichtigen;
die hämodynamischen Daten, die basierend auf der identifizierten Haltung erfasst wurden, durch Anwenden einer Einstellung der hämodynamischen Daten basierend auf der identifizierten Haltung zu normalisieren, um Vergleiche von hämodynamischen Daten zu erleichtern, die für verschiedene Haltungen erfasst wurden.

10. Computerlesbares Medium nach Anspruch 9, das ausgebildet ist, um die Effekte der Haltung des Patienten durch Speichern von hämodynamischen Druckdaten, die in einer Haltung erfasst wurden, getrennt von hämodynamischen Druckdaten, die in einer anderen Haltung erhalten wurden, zu berücksichtigen.

11. Computerlesbares Medium nach Anspruch 9, das ausgebildet ist, um die Änderung des erfassten hämodynamischen Drucksignals basierend auf einer Änderung des Drucks zu detektieren, die größer als ein vorbestimmter Druckschwellenwert ist.

12. Computerlesbares Medium nach Anspruch 11, wobei die Änderung des Druckes, die größer als ein vorbestimmter Druckschwellwert ist, auch innerhalb eines vorbestimmten Zeitintervalls aufzutreten hat.

13. Computerlesbares Medium nach Anspruch 12, wobei der vorbestimmte Druckschwellwert mindestens 5 mm Hg und das vorbestimmte Zeitintervall weniger als 5 Minuten beträgt.

## Revendications

1. Système de dispositif médical implantable (14) comportant :
des moyens (100) pour acquérir un signal de données hémodynamiques provenant d'un patient ;
un capteur de posture (120) adapté pour générer un signal de posture ;
des moyens (108) pour identifier une posture parmi au moins deux postures de patient à partir du signal de posture en un point donné dans le temps ;
des moyens (180) pour classer par catégories le signal de données hémodynamiques acquis en fonction de la posture identifiée ;
des moyens (102) pour mémoriser les données hémodynamiques classées par catégories, en sorte que les données hémodynamiques classées par catégories et mémorisées facilitent l'interprétation des données hémodynamiques mémorisées ;
**caractérisé en ce qu'**il comporte en outre :
des moyens de normalisation pour normaliser les données hémodynamiques acquises sur la base de la posture identifiée, les moyens de normalisation comportant des moyens pour appliquer un ajustement aux données hémodynamiques sur la base de la posture identifiée afin de faciliter des comparaisons de données hémodynamiques acquises dans différentes postures.

2. Système selon la revendication 1, dans lequel la mémorisation de données hémodynamiques basées sur la posture identifiée comporte la mémorisation de données hémodynamiques acquises dans une posture identifiée, séparément de données acquises dans d'autres postures identifiées.

3. Système selon la revendication 1, comportant en outre des moyens pour déterminer un pourcentage de temps passé dans chacune des postures identifiées.

4. Système selon la revendication 1, dans lequel les postures identifiées comportent une posture en supination et au moins une posture en décubitus latéral.

5. Système selon la revendication 1, comportant en outre un capteur d'activité (460) adapté pour générer un signal d'activité qui peut être utilisé pour identifier des changements de posture entre deux postures distinctes.

6. Système selon la revendication 5, dans lequel le signal d'activité peut être utilisé conjointement avec le signal de posture pour identifier des changements de posture entre deux postures couchées distinctes.

7. Système selon la revendication 6, dans lequel une augmentation temporaire dans le signal de capteur d'activité par rapport à un signal d'activité relativement faible est utilisée pour confirmer un changement de posture entre deux postures couchées distinctes.

8. Système selon la revendication 1, comportant en outre des moyens pour mesurer la fréquence cardiaque du patient, dans lequel une augmentation temporaire de la fréquence cardiaque mesurée par rapport à une fréquence relativement faible est utilisée pour confirmer un changement de posture entre deux postures couchées distinctes.

9. Support lisible par ordinateur comportant des instructions qui, lorsqu'elles s'exécutent sur un système tel que revendiqué dans l'une quelconque des revendications précédentes, amènent le système à :
acquérir un signal de pression hémodynamique en fonction du temps ;
détecter un changement dans le signal de pression hémodynamique acquis ;
associer le changement dans le signal de pression hémodynamique à un changement dans la posture du patient ;
mémoriser des données concernant le signal de pression hémodynamique et la posture du patient ; et
tenir compte des effets de la posture du patient dans la mémorisation du signal de pression hémodynamique ;
normaliser les données hémodynamiques acquises sur la base de la posture identifiée, en appliquant un ajustement aux données hémodynamiques basées sur la posture identifiée afin de faciliter des comparaisons de données hémodynamiques acquises dans différentes postures.

10. Support lisible par ordinateur selon la revendication 9, adapté pour tenir compte des effets de la posture du patient en mémorisant des données de pression hémodynamique obtenues dans une posture, séparément de données de pression hémodynamique obtenues dans une autre posture.

11. Support lisible par ordinateur selon la revendication 9, adapté pour détecter le changement dans le signal de pression hémodynamique acquis sur la base d'une variation de pression supérieure à un seuil de pression prédéterminé.

12. Support lisible par ordinateur selon la revendication 11, dans lequel la variation de pression supérieure à un seuil de pression prédéterminé doit également se produire sur un intervalle de temps prédéterminé.

13. Support lisible par ordinateur selon la revendication 12, dans lequel la variation de pression prédéterminé est d'au monis 5 mm de Hg et l'intervalle de temps prédéterminé est inférieur à 5 minutes
